# EUROPEAN PATENT APPLICATION

(11) **EP 4 198 143 A1**
(43) Date of publication of application: **21.06.2023**
(21) Application number: 22210751.8
(22) Date of filing: 01.12.2022
(51) Int. Cl.: C12Q 1/6816, C12Q 1/6853

(54) **MODIFIED PRIMERS FOR LOOP-MEDIATED ISOTHERMAL AMPLIFICATION AND USE THEREOF**

(30) Priority: 14.12.2021 KR 20210178467; 18.11.2022 KR 20220155260
(71) Applicant: 1Drop Inc., Gyeonggi-do 13217 (KR)
(72) Inventor: HONG, Seong Soo, 12779 Gyeonggi-do (KR); PARK, Jeong Ho, 13011 Gyeonggi-do (KR); RHEE, Joo Won, 13562 Gyeonggi-do (KR)
(74) Representative: advotec.

(57) **Abstract**

The present invention relates to an inner primer which includes, in the 3'-end direction, a blocker for inhibiting gene amplification and an RNA sequence that is complementary to a template target nucleic acid sequence and is cleaved by a ribonuclease; a primer composition and a kit including the same; and a method for detecting a target nucleic acid sequence by using the same. The present invention can increase amplification efficiency and detection specificity by inhibiting non-specific binding during an amplification process.

## Description

### CROSS-REFERENCE TO RELATED APPLICATIONS

This application claims the priority of Korean Patent Application No.10-2022-0155260 filed on November 18, 2022, in the Korean Intellectual Property Office, the disclosure of which is incorporated herein by reference.

### BACKGROUND

### Field

The present invention relates to a primer, a composition, a kit for looped-mediated isothermal amplification of a target nucleic acid sequence, and a method for detecting the target nucleic acid sequence.

### Description of the Related Art

The looped-mediated isothermal amplification (LAMP) has an advantage of amplifying a gene at the same temperature and thus can be widely used in the field of Point of care testing (POCT) as compared to a polymerase chain reaction (PCR) method using various temperatures.

In the LAMP, a double-stranded chain-specific intercalator such as SYBR Green may be added to measure fluorescence intensity in real time as nucleic acid amplification products increase, or color change may be measured in real time through a pH indicator of pH that changes by DNA polymerase during gene amplification. However, the measurement method using the SYBR Green, and the pH indicator does not specifically identify the sequence of the nucleic acid amplification product, and in the case of the LAMP, since four to six primers are used for one target gene amplification, a gene amplification dimer is generated by a bond between primers, and thus there is a possibility that a target reaction is inhibited and a false positive may occur. In addition, in order to distinguish the non-specific reaction, after the real-time measurement, the temperature is increased to 95°C to measure the annealing temperature of the nucleic acid amplification product to determine whether there is a non-specific reaction, thereby making it difficult to perform multiple detection.

Thus, as a method of excluding a structure in which a dimer may occur, software designating a primer sequence for looped-mediated isothermal gene amplification has been developed, but there is a limitation in that the *in vitro* result cannot be completely predicted and the desired gene region cannot be accurately amplified. In particular, during multiple gene amplification, a gene amplification problem due to the binding between primers is likely to occur, and a method for improving this is required.

### SUMMARY

The present invention provides a primer for looped-mediated isothermal amplification.

Another object of the present invention is to provide a primer composition for looped-mediated isothermal amplification.

Another object of the present invention is to provide a kit for looped-mediated isothermal amplification.

Still another object of the present invention is to provide a method for detecting a target nucleic acid sequence using a primer composition for looped-mediated isothermal amplification.

In order to accomplish the above objects, the present invention provides an inner primer for the looped-mediated isothermal amplification (LAMP), a primer composition including the same, and a kit including the composition, in which the inner primer may be a forward inner primer (FIP), a backward inner primer (BIP), or a combination thereof, and the FIP, BIP, or combination thereof includes an RNA sequence that is complementary to the template target nucleic acid sequence in a 3'-end direction and is cleaved by Ribonuclease (RNase) and a blocker for inhibiting gene amplification.

In order to achieve the above objects, the present invention provides a method for detecting a target nucleic acid sequence, the method including performing looped-mediated isothermal amplification on a template target nucleic acid sequence using the primer composition for looped-mediated isothermal amplification.

According to the present invention, the amplification efficiency and the detection specificity can be increased by identifying individual gene amplification signals without a separate probe in the multiple-gene amplification and inhibiting non-specific binding and dimer formation in the amplification process, by including a blocker for inhibiting gene amplification and an RNA sequence that is complementary to a template target nucleic acid sequence in the 3'-end direction of the inner primer and is cleaved by a ribonuclease.

### BRIEF DESCRIPTION OF THE DRAWINGS

The above and other aspects, features and other advantages of the present disclosure will be more clearly understood from the following detailed description taken in conjunction with the accompanying drawings, in which:

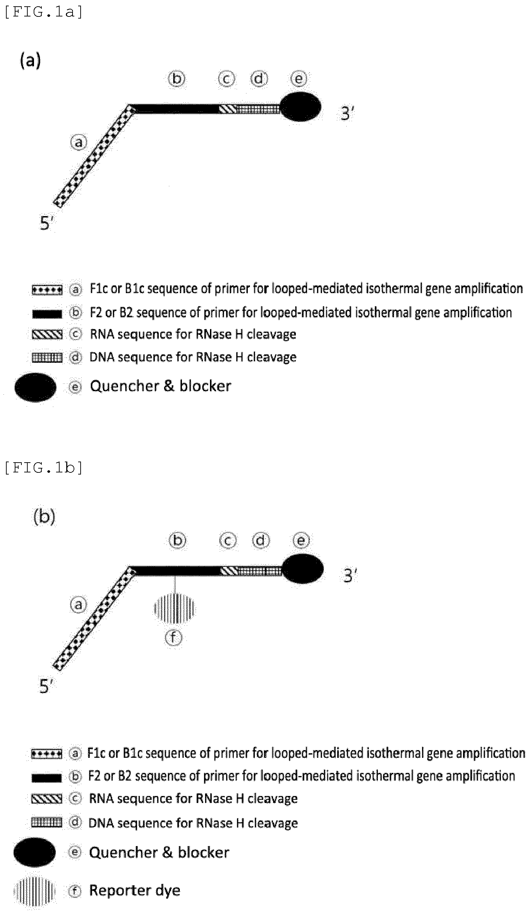

FIG. 1A illustrates an HSIA primer structure according to an example, which is a [F1C]-[F2]-[RNA sequence for RNase cleavage]-[DNA sequence for RNase cleavage]-blocker/quencher structure from 5' to 3'-end direction of a forward inner primer (FIP), or a [B1C]-[B2]-[RNA sequence for RNase cleavage]-[DNA sequence for RNase cleavage]-blocker/quencher structure from 5' to 3' end direction of a backward inner primer (BIP). Here, a is the base sequence that forms a loop with the F1c sequence of the FIP or the B1c sequence of the BIP, and b is the base sequence that is complementary to the target nucleic acid with the F2 sequence of the FIP or the B2 sequence of the BIP. c is an RNA sequence hydrolyzed by RNase and is complementary to the target template nucleic acid, and d is a DNA sequence that enhances the recognition efficiency of RNase and is complementary to the target template nucleic acid. e is a blocker that inhibits gene amplification, and f is a reporter dye. FIG. 1B illustrates an HSIA primer structure according to another example in which a reporter dye is added to the HSIA primer of FIG. 1A, that is, an [F1C]-[F2 to which the reporter dye is attached]-[RNA sequence for RNase cleavage]-[DNA sequence for RNase cleavage]-blocker/quencher structure from a 5' to 3'-end direction of a forward inner primer (FIP), or an [B1C]-[B2 to which the reporter dye is attached]-[RNA sequence for RNase cleavage]-[DNA sequence for RNase cleavage]-blocker/quencher structure from a 5' to 3'-end direction of a backward inner primer (BIP);

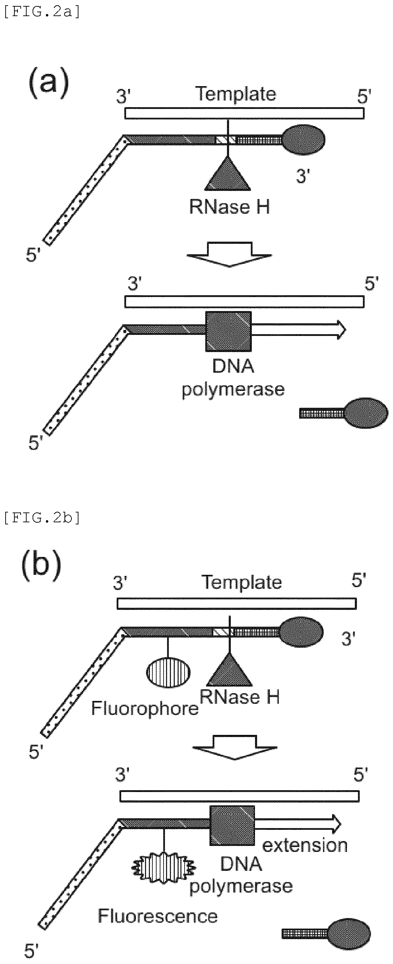

FIG. 2A shows the HSIA primer structure of FIG. 1A and a process in which RNase H is elongated by DNA polymerase after DNA-RNA binding is cleaved, and FIG. 2B shows the HSIA primer structure of FIG. 1B and a process in which RNase H is elongated and unquenched by DNA polymerase after DNA-RNA binding is cleaved, thereby expressing fluorescence;

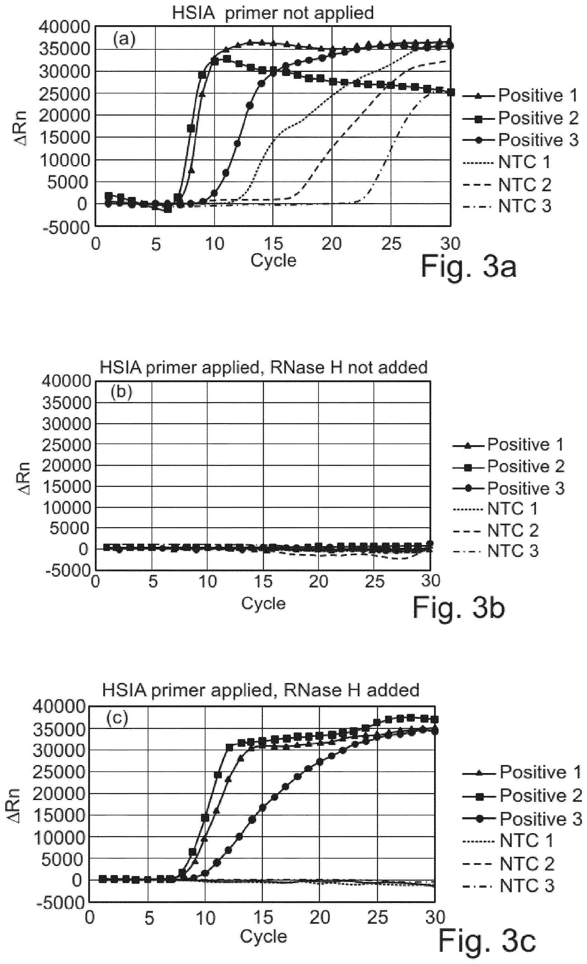

FIG. 3 shows the effect of improving the specificity of the single looped-mediated isothermal gene amplification method when HSIA primer is applied in fluorescence measuring equipment, in which FIG. 3A shows a condition in which HSIA primer is not applied, FIG. 3B shows a condition in which HSIA primer is applied and RNase H is not added, and FIG. 3C shows a result of confirming SYBR green signal when HSIA primer is applied and RNase H is added;

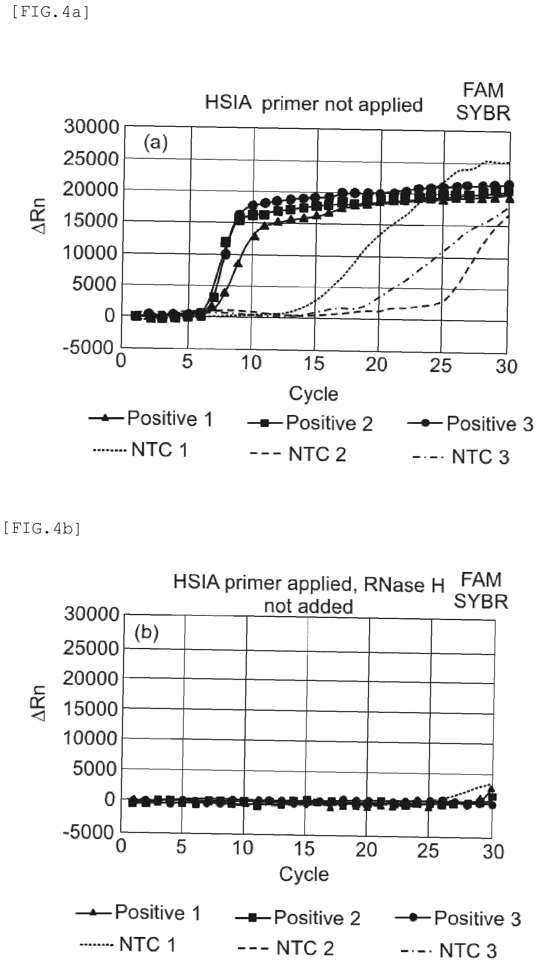

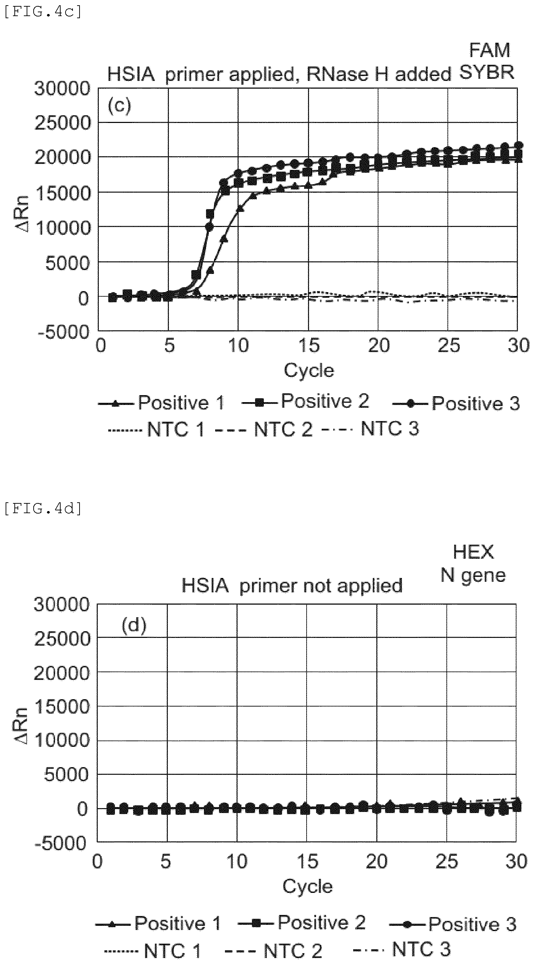

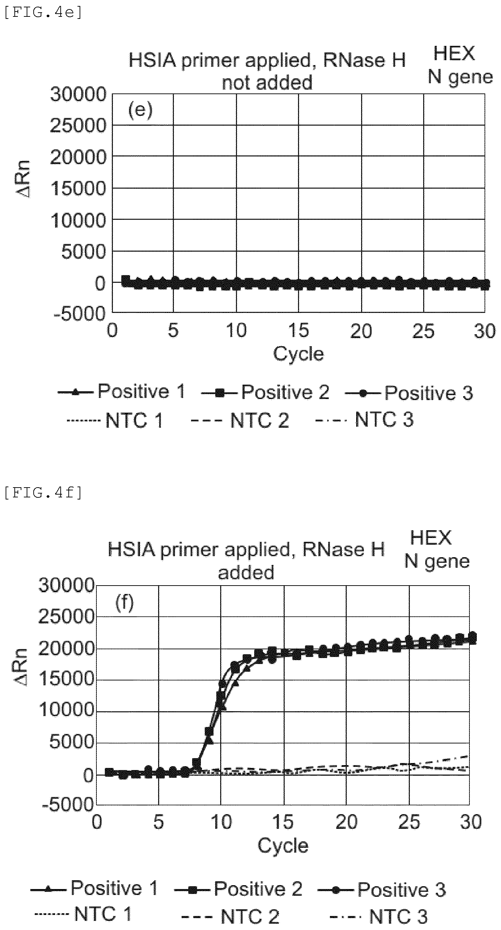

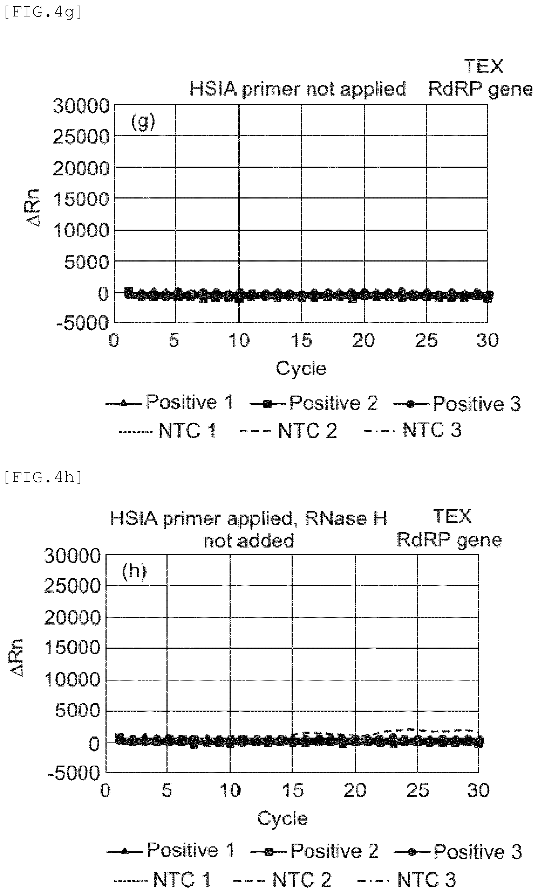

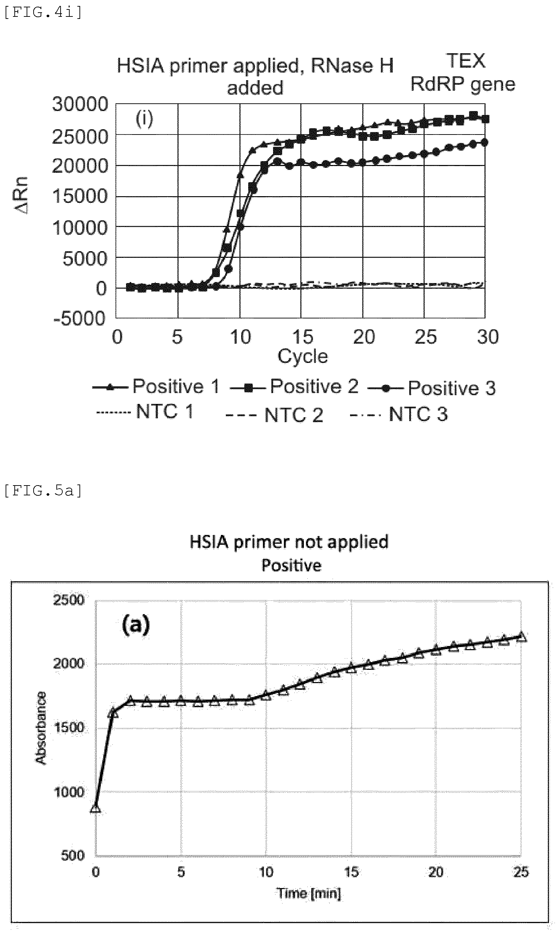

FIG. 4 shows the effect of improving the specificity of the multiple-looped-mediated isothermal gene amplification method and the ability to detect multiple targets when an HSIA primer is applied in fluorescence measuring equipment, and FIG. 4A shows the result of confirming an FAM fluorescence signal under the condition of not applying an HSIA primer, FIG. 4B shows the result of confirming an FAM fluorescence signal under the condition of applying an HSIA primer and not adding an RNase H, and FIG. 4C shows the result of confirming an FAM fluorescence signal under the condition of applying an HSIA primer and adding RNase H. FIGS. 4E and 4H show results of identifying fluorescence signals of HEX and Texas Red channels under the conditions of application of HSIA primer and non-addition of RNase H, FIG. 4F shows results of identifying fluorescence signals of HEX channels under the conditions of application of HSIA primer and addition of RNase H, and FIG. 4I shows results of identifying fluorescence signals of Texas Red channels under the conditions of application of HSIA primer and addition of RNase H; and

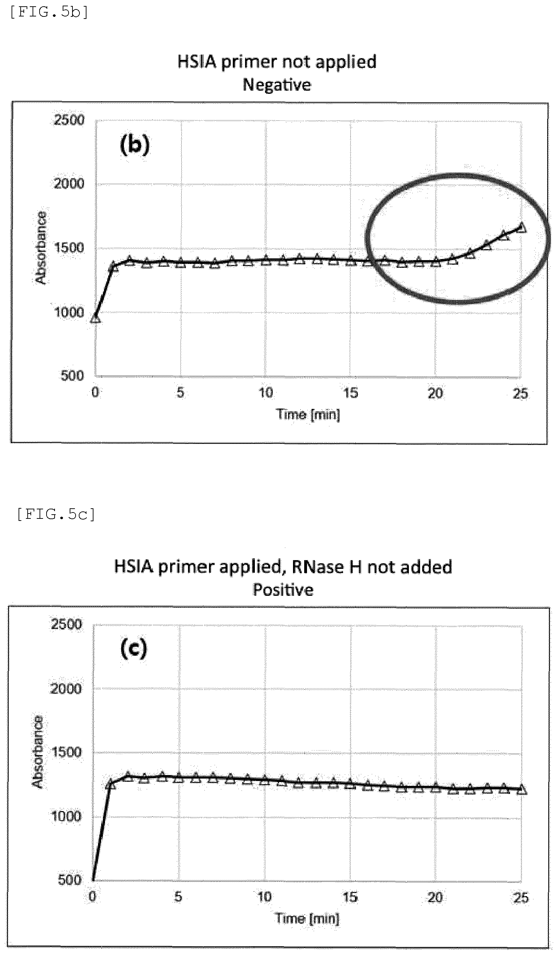

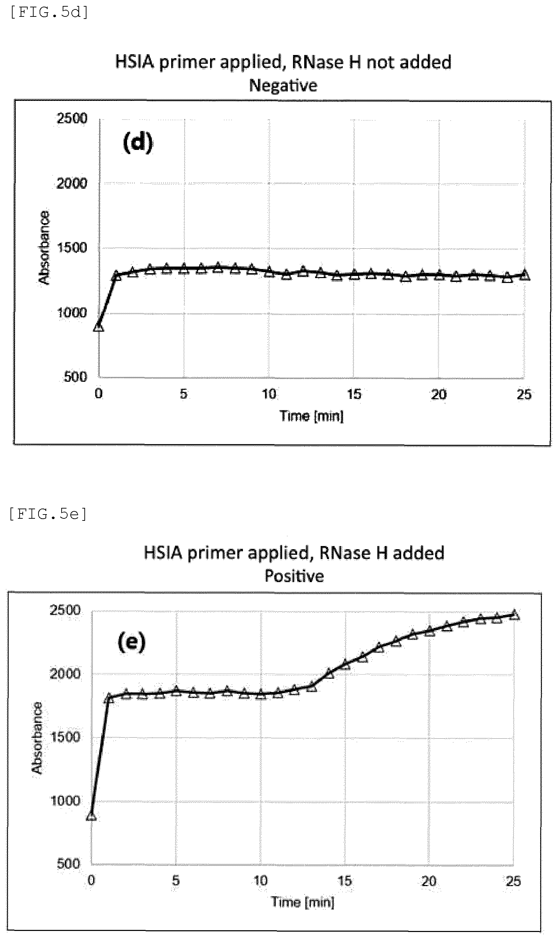

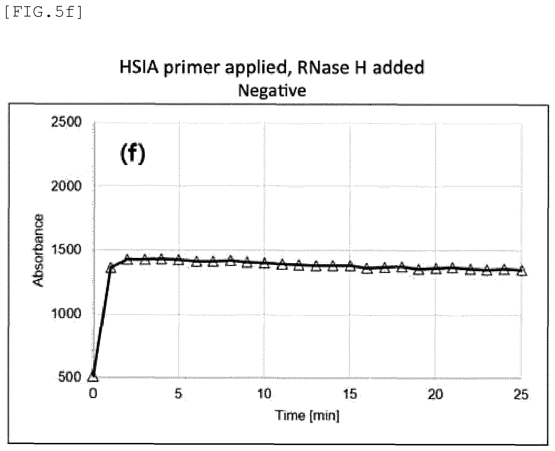

FIG. 5 shows the effect of improving the specificity of the looped-mediated isothermal gene amplification method when the HSIA primer is used in the absorbance measuring equipment, and FIGS. 5A and 5B show the results of confirming absorbance when the HSIA primer is not applied, FIGS. 5C and 5D show the results of confirming absorbance when the HSIA primer is applied and the RNase H is not added, and FIGS. 5E and 5F show the results of confirming absorbance when the HSIA primer is applied and the RNase H is added.

### DETAILED DESCRIPTION OF THE EMBODIMENT

The present invention provides a modified inner primer for the looped-mediated isothermal amplification (LAMP) of a target nucleic acid sequence, in which the inner primer may be a forward inner primer (FIP) or a backward inner primer (BIP) or a combination thereof, and the FIP, the BIP or the combination thereof includes a blocker which inhibits gene amplification and an RNA sequence which is complementary to a template target nucleic acid sequence in a 3'-end direction and is cleaved by Ribonuclease (RNase).

The term "amplification" as used herein refers to an increase in the number of copies of a nucleic acid molecule, such as a gene or a fragment of a gene. The amplified product may also be referred to as an amplification product. An example of *in vitro* amplification may include polymerase chain reaction (PCR) in which a sample (e.g., a biological sample) is brought into contact with a pair of oligonucleotide primers under conditions that permit hybridization of the primer to a nucleic acid molecule in the sample. Further, other examples of *in vitro* amplification may include real-time PCR, quantitative real-time PCR, reverse transcription PCR (RT-PCR), quantitative RT-PCR (qRT-PCR), looped-mediated isothermal amplification (LAMP); reverse-transcription LAMP (RT-LAMP); strand displacement amplification (SDA) (U.S. Pat. No. 5,744,311; and the like).

The term "Looped-mediated isothermal amplification (LAMP)" is a method capable of performing an amplification reaction under a condition of isothermal temperature, unlike the existing polymerase chain reaction (PCR). While the conventional PCR should be accompanied by a temperature change through three steps of denaturation, annealing, and extension, the looped-mediated isothermal amplification is a molecular biological technique that enables annealing and extension at a constant temperature without such a temperature change to replicate and amplify a desired portion of a target sequence. Since the isothermal amplification reaction uses Bst DNA polymerase, etc. having a strand displacement function instead of Taq DNA polymerase used in conventional PCR, annealing and extension are performed at a fixed temperature close to the Tm value without depending on heat. Therefore, unlike PCR, the isothermal amplification reaction can amplify genes without specialized equipment and has the advantage of being able to amplify genes at high concentrations within a short time.

In general, the LAMP method uses four or more primers specific for the six regions in the target nucleic acid sequence. The primers include a forward outer primer (F3), a backward outer primer (B3), a forward inner primer (FIP), and a backward inner primer (BIP). The four primers are primers used in the LAMP method (Notomi et al., Nucl. Acids. Res. 28:E63, 2000; Nagamine et al., Mol. Cell. Probes 16:223-229, 2002; Mori et al., J. Biochem. Biophys. Methods 59: 145-157, 2004), which is well known to those skilled in the art. Also, in order to increase amplification efficiency or reaction rate, a forward loop primer (Loop F: LF) and a backward loop primer (Loop B: LB) may be further included. The external primers serve to release the DNA double strand during the non-cyclic step of the reaction. The inner primer is composed of nucleic acids corresponding to the forward and backward base sequences so as to form a loop that is essential for looped-mediated isothermal amplification. Additional loop primers attach to the base sequence to which the inner primer does not bind to accelerate the looped-mediated isothermal amplification reaction.

The four primers are designed to have a sequence corresponding to the sequence of six regions, i.e., a B3 region, a B2 region, a B1 region, an F1c region, an F2c region, or an F3c region on the target nucleic acid, or to have a sequence corresponding to the sequence of an F3 region, an F2 region, an F1 region, a B1c region, a B2c region, and a B3c region. The six regions may be located on a single-stranded target nucleic acid in the order of [B3]-[B2]-[B1]-target region-[F1c]-[F2c]-[F3c] from 5' to 3'-end direction, or [F3]-[F2]-[F1]-target region-[B1c]-[B2c]-[B3c] from 5' to 3' end direction. The four primers may be used to amplify a target nucleic acid sequence including [B3]-[B2]-[B1]-target region-[F1c]-[F2c]-[F3c] from a 5' to 3'-end direction or [F3]-[F2]-[F1]-target region-[B1c]-[B2c]-[B3c] from a 5' to 3'-end direction. The four primers may be used to amplify a target region.

Here, F1, F1c, F2, F2c, F3, F3c, B1, B1c, B2, B2c, B3, and B3c may refer to or describe a specific region on a target nucleic acid, a sequence of the specific region, the primer itself, or a sequence of the primer, and the lower case "c" refers to a complementary sequence. For example, F3 includes a sequence that can anneal to the sequence of the F3c region having a sequence complementary to that of the F3 region, B3 includes a sequence that can anneal to the sequence of the B3c region having a sequence complementary to that of the B3 region, FIP includes an F1c sequence that can anneal to the sequence of the F1 region from 5' to 3' end direction and an F2 sequence that can anneal to the sequence of the F2c region, and BIP includes a B1c sequence that can anneal to the sequence of the B1 region from 5' to 3' end direction and a B2 sequence that can anneal to the B2c sequence. The LAMP reaction produces DNA having a stem and loop structure with an inverted repeat of the target nucleic acid sequence.

The target nucleic acid, the target nucleic acid sequence, the target gene, the target gene sequence, or the target sequence refers to a nucleic acid sequence of interest for detection or quantification. The nucleic acid sequence may include a single strand as well as a double strand sequence. The nucleic acid sequence may include a sequence newly generated in the reaction as well as a sequence initially present in a nucleic acid sample.

The target nucleic acid may include any DNA (gDNA and cDNA), RNA, or a hybrid thereof (chimeric nucleic acid). The target nucleic acid may have a double-stranded or single-stranded form.

The target nucleic acid may include any natural prokaryotic nucleic acid, eukaryotic nucleic acid (e.g., protozoan and parasites, fungi, yeast, higher plants, lower and higher animals including mammals and humans), virus (e.g., herpes virus, HIV, influenza virus, Epstein-Barr virus, hepatitis virus, polio virus, etc.) nucleic acid, or viroid nucleic acid. In addition, the nucleic acid molecule may be any nucleic acid molecule that is recombinantly produced or may be produced, or that is chemically synthesized or may be synthesized. Thus, nucleic acid molecules may be found or not found in nature. Here, the target nucleic acid sequence may include a known or unknown sequence.

The term "primer" refers to a short nucleic acid sequence that can be base-paired with a complementary template with a nucleic acid sequence having a short free 3'-end hydroxyl group and serves as a starting point for copying the template strand. The primer may be annealed to the complementary target nucleic acid strand by nucleic acid hybridization and extended along the target nucleic acid strand by a DNA polymerase. The length and sequence of the primers are combined to initiate repeated superposition synthesis of the gene to be amplified, which can be appropriately adjusted according to the ionic strength and maintenance conditions at isothermal temperature, as well as the complexity of the desired DNA or RNA target. The primer may be, for example, 10 to 60, 15 to 60, 15 to 50, 15 to 45, 15 to 40, 20 to 40, 20 to 50, 25 to 50, 25 to 60, or 30 to 60 bp.

The primer may be chemically synthesized using a phosphoramidite solid support method or other well-known methods. Such nucleic acid sequences may also be modified using a number of means known in the art. Non-limiting examples of such modifications include methylation, capsulation, substitution of natural nucleotides into onr or more homologues, and modifications between nucleotides, such as modifications to uncharged linkages (e.g., methylphosphonate, phosphotriester, phosphoroamidate, carbamate, etc.) or charged linkages (e.g., phosphorothioate, phosphorodithioate, etc.). The nucleic acid may be one that contains one or more additional covalently linked residues, such as a protein (e.g., nuclease, toxin, antibody, signal peptide, poly-L-lysine, etc.), intercalating agent (e.g., acridine, psoralen, etc.), chelating agent (e.g., metal, radioactive metal, iron, oxidizable metal, etc.), or alkylating agent. In addition, the primer may be modified by using a marker which may directly or indirectly provide a detectable signal. Examples of the marker include radioactive isotopes, fluorescent molecules, biotin, and the like.

The term "complementary sequence" as used herein refers to a nucleic acid sequence in which bases of single-stranded nucleic acids form a matching base pair to stably form a double-stranded structure. Such a complementary sequence may be included in the complementary sequence if some matching of the base sequence, e.g., 99%, 98%, 97%, 96%, 95%, 94%, 93%, 92%, 91%, 90%, 89%, 87%, 86%, 85%, 84%, 83%, 82%, 81%, 80%, 79%, 78%, 77%, 76%, or 75%, as well as 100% matching base sequences can stably form a double-stranded structure.

The term "annealing" refers to a process in which a single-stranded DNA or RNA molecule having a complementary base sequence forms a stable double-stranded nucleic acid molecule by hydrogen bonding between base pairs.

The FIP, BIP, or a combination thereof includes an RNA sequence that is complementary to a template target nucleic acid sequence in the 3'-end direction and is cleaved by a Ribonuclease (RNase) and a blocker that inhibits gene amplification.

The RNA sequence induces DNA-RNA binding cleavage or degradation of RNase. The RNA sequence may be a sequence at a position where looped-mediated isothermal amplification is maintained and possible even if it is removed by RNase. The RNA sequence cleaved by the RNase is complementary to the template target nucleic acid and may be 1 to 20, 1 to 15, 1 to 10, 2 to 8, or 2 to 6 bp, but is not limited thereto.

The RNase may be RNase H, RNase A, or RNase R1, for example, RNase H. RNase H refers to an RNase that specifically degrades only the RNA strand of a hybrid double strand of RNA and DNA.

The blocker inhibits DNA polymerization or amplification of a target sequence, and may be an abasic spacer in which a base cannot be aligned. The blocker may be a non-base ribonucleic acid spacer (rSpacer, rSp), a non-base nucleic acid spacer (dSpacer), a C3 spacer, or a C6 spacer that does not include a base, or may be any spacer modification that does not include a base. Alternatively, the blocker may be a mismatch sequence. The mismatch sequence may be adenine, guanine, cytosine, thymine, uridine, or modified therefrom, which is not complementary to the target nucleic acid.

The blocker may include a quencher.

The RNA sequence cleaved by the RNase may include a reporter in a 5'-end direction. The reporter refers to selectable markers for notifying the presence, location, or level of a target.

The reporter may be located in the direction of the 5' end of the RNA sequence cleaved by RNase, or may be located between 1 to 100, 2 to 85, 5 to 75, 10 to 60, 15 to 50, or 20 to 30 bp in the direction of the 5' end of the RNA sequence cleaved by RNase, but may not be limited thereto. The reporter may be located in any region of FIP, BIP, or a combination thereof, or any region of F2, B3, or a combination thereof, but is not limited thereto.

When the RNA strand is degraded or cleaved in DNA-RNA binding, unquenching may be induced as the sequence including the quencher falls off the template DNA. The reporter and the quencher may be donors and acceptors.

The reporter and quencher may include any molecule known in the art. For example, the reporter and quencher molecules may be selected from the group consisting of Cy2^{™}(506), YO-PRO^{™}-1(509), YOYO^{™}-1(509), Calcein(517), FITC(518), FluorX^{™}(519), Alexa^{™}(520), Rhodamine 110(520), Oregon Green^{™} 500(522), Oregon Green^{™} 488(524), RiboGreen^{™}(525), Rhodamine Green^{™}(527), Rhodamine 123(529), Magnesium Green^{™}(531), Calcium Green^{™}(533), TO-PRO^{™}-1(533) , TOTO1(533), JOE(548), BODIPY530/550(550), Dil(565), BODIPY TMR(568), BODIPY558/568(568), BODIPY564/570(570), Cy3^{™}(570), Alexa^{™} 546(570), TRITC(572), Magnesium Orange^{™}(575), Phycoerythrin R&B(575), Rhodamine Phalloidin(575), Calcium Orange^{™}(576), Pyronin Y(580), Rhodamine B(580), TAMRA(582), Rhodamine Red^{™}(590), Cy3.5^{™}(596), ROX(608), Calcium Crimson^{™}(615), Alexa^{™} 594(615), Texas Red(615), Nile Red(628), YO-PRO^{™}-3(631), YOYO^{™}-3(631), R-phycocyanin(642), C-Phycocyanin(648), TO-PRO^{™}-3(660), TOTO3(660), DiD DilC(5) (665), Cy5^{™}(670), Thiadicarbocyanine(671), Cy5.5(694), HEX(556), TET(536), Biosearch Blue(447), CAL Fluor Gold 540(544), CAL Fluor Orange 560(559), CAL Fluor Red 590(591), CAL Fluor Red 610(610), CAL Fluor Red 635(637), FAM(520), Fluorescein(520), Fluorescein-C3(520), Pulsar 650(566), Quasar 570(667), Quasar 670(705), Quasar 705(610), DABCYL, and BHQ (Black Hole Quencher). For example, the reporter may be FAM, TET, HEX, TAMRA, ROX, Texas Red, Cy3, or Cy5 as a dye, and the quencher may be TAMRA, DABCYL, BHQ 1, or BHQ 2. Multiplex target sequence amplification is possible according to the reporter and quencher molecules.

The reporter or quencher may modify a sequence to an internal dT or internal dG, an internal dC, an internal dA, an internal dU, or an internal dR (e.g., modify thymine to internal dT), and place the reporter or quencher at the site. In this case, the entire or partial sequence forms a double strand at a temperature equal to or lower than a certain temperature.
- Internal dT: Internal Amino-C6(or C2)-deoxythymine
- Internal dA: Internal Amino-C6-deoxyadenocine
- Internal dC: Internal Amino-C6-deoxycytidine
- Internal dG: Internal Amino-C6-deoxyguanosine
- Internal dU: Internal Amino-C6-deoxyuridine
- Internal dR: Internal Amino-C6-deoxyribose

The FIP, the BIP, or a combination thereof may include a DNA sequence that is complementary to the template target nucleic acid sequence in the 3'-end direction and recognized by the RNase. The DNA sequence increases the recognition efficiency of RNase. The DNA sequence recognized by the RNase is complementary to the template target nucleic acid and may be 1 to 20, 1 to 15, 1 to 10, 2 to 8, or 3 to 6 bp, but is not limited thereto.

The primer(s) according to the present invention may be referred to as a High specific isothermal amplification technology (HSIA) primer.

The present invention also provides a primer composition for the looped-mediated isothermal amplification (LAMP) including an inner primer for the LAMP, a forward outer primer (F3), and a backward outer primer (B3). The F3 and B3 may be template target nucleic acids and those designed according to the above descriptions. The composition may include Ribonuclease (RNase).

The present invention also provides a kit for the looped-mediated isothermal amplification (LAMP) including the composition.

The kit may further include a DNA polymerase in the case of the LAMP reaction or a reverse transcriptase and a DNA polymerase in the case of the RT-LAMP reaction. The LAMP may be a LAMP reaction when the template nucleic acid is a DNA molecule or an RT-LAMP reaction when the template nucleic acid is an RNA molecule. The DNA polymerase used in the LAMP reaction or the RT-LAMP reaction is not limited as long as it can be used in the LAMP. The DNA polymerase that can be used in the LAMP or RT-LAMP reaction may be a DNA polymerase having a high strand displacement activity, for example, a DNA polymerase long fragment (LF) of thermophilic bacteria such as *Bacillus stearothermophilus* (Bst), *Bacillus Smithii* (Bsm), *Geobacillus* sp. M (GspM), or *Thermodesulfatator indicus* (Tin), or a variant thereof, or a Taq DNA polymerase variant. The reverse transcriptase used in the RT-LAMP reaction is not limited as long as it can be used for the RT-LAMP. For example, the reverse transcriptase may be derived from Avian myeloblastosis virus (AMV) or moloney murine leukemia virus (MMLV).

The kit may further include additional reagents such as a solution including ATP, CTP, GTP, and UTP, a solution including dNTPs, and/or a reaction buffer for a transcription reaction. The kit may include salts, preservatives, enzymes, and the like commonly used for target sequence amplification. The additional reagents may be contained in the same container together with the primer composition, or may be contained separately in a separate container.

The present invention also provides a method for detecting a target nucleic acid sequence, the method including the steps of: (a) selecting a sequence of six regions of B3, B2, B1, F1c, F2c, and F3c or a sequence of six regions of F3, F2, F1, B1c, B2c, and B3c from 5' to 3' end direction of the target nucleic acid sequence; and (b) performing the looped-mediated isothermal amplification (LAMP) on a sample using the primer composition and a Ribonuclease (RNase).

The sequences of the six regions of B3, B2, B1, F1c, F2c, and F3c, the nucleic acid sequences of the six regions of F3, F2, F1, B1c, B2c, and B3c, and the primer composition are the same as described above.

The looped-mediated isothermal amplification may be performed in a buffer or reaction solution at 40 to 80 degrees, 45 to 75 degrees, or 55 to 70 degrees, for 1 to 120 minutes, 2 to 90 minutes, 3 to 75 minutes, 4 to 60 minutes, 5 to 40 minutes, 7 to 30 minutes, 8 to 20 minutes, or 8 to 15 minutes. For example, the fluorescence may be measured at 50 to 60 degrees or 52 to 57 degrees for annealing and extension for 1 to 10 minutes or 2 to 7 minutes, at 65 to 75 degrees or 67 to 72 degrees for 1 to 10 minutes or 2 to 7 minutes to activate an enzyme inactivated by an antibody, etc., and at 55 to 65 degrees or 57 to 62 degrees for 20 to 40 minutes or 25 to 35 minutes.

When the RNase is extended and amplified by a DNA polymerase after RNA is cleaved, and additionally, when the amount of luminescence or fluorescence is measured by unquenching, non-specific binding and dimer formation are suppressed in an amplification process, thereby increasing amplification efficiency and detection specificity.

The sample may be obtained from a subject such as a human or an animal other than human. The sample may include cells, tissues, or fluids, and may include, but is not limited to, viruses, bacteria, tissues, cells, blood, serum, plasma, lymph, urine, feces, eye fluids, saliva, sputum, semen, brain extracts, spinal cord fluid, appendix, bronchoalveolar lavage fluid, spleen and tonsillar tissue extracts, amniotic fluid, ascites, hair roots, and non-biological samples (e.g., food, water, and soil). Also, the sample may include a natural nucleic acid molecule and a synthetic nucleic acid molecule isolated from a biological source.

According to the method, the presence and level of a target nucleic acid may be identified by isothermally amplifying the target nucleic acid, and thus disease diagnosis and prognosis prediction such as infectious diseases, and genetic diseases, treatment reactivity, drug resistance, drug tolerance, and the like may be identified.

Hereinafter, the present invention will be described in detail through embodiments. However, the following embodiments are only for illustrating the present invention, and the contents of the present invention are not limited by the following embodiments.

### Example 1. Confirmation of specificity improvement when using primer including RNA sequence and blocker in looped-mediated isothermal amplification (LAMP) using fluorescence measuring real-time gene amplification device and intercalating dye

In order to solve a problem in which a non-specific signal is generated due to the formation of a primer dimer in a conventional LAMP, it was confirmed whether detection specificity is improved when a HSIA primer including a blocker and an RNA sequence complementary to a template nucleic acid is used.

Targets and primers were designed with the sequence of the N gene site using the sequence of the SARS-CoV-2 sequence (GenebankID: NC_045512.2).

**[Table 1]**

| **Primer** | **SEQ ID NO.** | **SEQ ID NO: 5' -> 3'** |
|---|---|---|
| N_F3 primer | SEQ ID NO: 1 | TGGCTACTACCGAAGAGCT |
| N_B3 primer | SEQ ID NO: 2 | TGCAGCATTGTTAGCAGGAT |
| N_FIP primer | SEQ ID NO: 3 | TCTGGCCCAGTTCCTAGGTAGTGACGAATTCGTGGTGGTGA |
| N_BIP primer | SEQ ID NO: 4 | AGACGGCATCATATGGGTTGCACGGGTGCCAATGTGATCT |
| N_LF primer | SEQ ID NO: 5 | GAAATACCATCTTGGACTGAGATC |
| N_LB primer | SEQ ID NO: 6 | TGAGGGAGCCTTGAATACAC |

Modified HSIA primers were designed for the N_FIP and N_BIP primers. At the 3' end of N_FIP (SEQ ID NO: 3) and N_BIP (SEQ ID NO: 4), RNA sequence 3 bp complementary to the SARS-CoV-2 sequence was added, and complementary DNA sequence 4 bp was added so that the blocker was removed by RNase H when the target gene was present. In order to prevent primer dimer formation in the added sequence, a C3 blocker was added to the 3' end of N_FIP and N_BIP to design primers of N_FIP_HSIA (SEQ ID NO: 7) and N_BIP_HSIA (SEQ ID NO: 8).

**[Table 2]**

| **Primer** | **SEQ ID NO.** | **SEQ ID NO: 5' -> 3' (r mark corresponds to RNA sequence)** |
|---|---|---|
| N_FIP_HSIA primer | SEQ ID NO: 7 | TCTGGCCCAGTTCCTAGGTAGTGACGAATTCGTGGTGGTGArCrGrGTA AA-C3 |
| N_BIP_HSIA primer | SEQ ID NO: 8 | AGACGGCATCATATGGGTTGCACGGGTGCCAATGTGATCTrUrUrUGGT G-C3 |

In order to confirm the difference between the positive amplification signal and the non-specific amplification signal in the real-time gene amplification device when the HSIA primer was not applied, 1.6 µM of each of 1× isothermal amplification buffer 2 (NEB, USA), 0.1% v/v Tween 20, 8 mM MgSO₄, and 1.4 mM of each of dNTP, BST 3.0 polymerase 5U (NEB, USA), and SYBR green 1× (Biorad, USA), , and FIP and BIP primers (SEQ ID NOs: 3 and 4) to which the HSIA primer was not applied, 0.2 µM of each of F3 and B3 primers (SEQ ID NOs: 1 and 2), and 0.4 µM of each of LF and LB primers (SEQ ID NOs: 5 and 6) were used.

The positive amplification signal was confirmed by using an Accuplex^{™} SARS-CoV-2 reference/standard material (Seracare, USA) at a concentration of 1 × 10^{^5} copies/rxn and adding Diethylpyrocarbonate(DEPC)-treated distilled water (DEPC-treated DW) (Geneall, Korea) as a negative control group to confirm an amplification signal by a dimer. For the nucleic acid amplification conditions, after treatment by ABI7500 real-time PCR (Thermofisher, USA) equipment for 5 minutes at 55°C and 5 minutes at 70°C, the fluorescence was measured at 60°C for 30 minutes.

In order to confirm the specificity improvement effect when HSIA primer was applied, 1.6 µM of each of 1× isothermal amplification buffer 2 (NEB, USA), 0.1% v/v Tween 20, 8 mM MgSO₄, and 1.4 mM of each of dNTP, BST 3.0 polymerase 5U (NEB, USA), and SYBR green 1× (Biorad, USA), , and FIP and BIP HSIA primers (SEQ ID NOs: 7 and 8), 0.2 µM of each of F3 and B3 primers (SEQ ID NOs: 1 and 2), 0.4 µM of each of LF and LB primer (SEQ ID NOs: 5 and 6) were used.

In order to confirm a blocker removal effect in the presence of a target nucleic acid by RNase H, two conditions, i.e., a condition using 6 ng/ml Hot start RNase H (1drop, Korea) and a condition not using the same, were tested. The positive signal was confirmed by using an Accuplex^{™} SARS-CoV-2 reference/standard material (Seracare, USA) at a concentration of 1 × 10^{^5} copies/rxn, and adding Diethylpyrocarbonate(DEPC)-treated distilled water (DEPC-treated DW) (Geneall, Korea) as a negative control group to confirm an amplification signal by a dimer. For the nucleic acid amplification conditions, after treatment by ABI7500 real-time PCR (Thermofisher, USA) equipment for 5 minutes at 55°C and 5 minutes at 70°C, the fluorescence was measured at 60°C for 30 minutes.

FIG. 3 shows the effect of improving the specificity of the single-looped-mediated isothermal gene amplification method when HSIA primer was applied in fluorescence measuring equipment, and in FIG. 3A, as a result of confirming the SYBR green signal under the condition in which HSIA primer was not applied, the amplification signal of the positive standard material was confirmed to be 7 to 11 minutes, and the amplification signal of the negative control group (DW) was confirmed to be 13 to 24 minutes. In FIG. 3B, as a result of the HSIA primer application and the RNase H non-addition test, when both the positive amplification signal and the negative control amplification signal were not confirmed and thus the blocker was not removed, the positive amplification signal and the non-specific amplification signal by the primer dimer were not confirmed. In FIG. 3C, as a result of the HSIA primer application and the RNase H addition test, the amplification signal in the positive standard material was confirmed to be 8 to 11 minutes, but the amplification signal was not confirmed in the negative control group. Therefore, according to the results of FIGS. 3A, 3B, and 3C, non-specific amplification by the primer dimer was improved upon application of the HSIA primer, and positive signal amplification of the positive standard material was confirmed.

### Example 2. Confirmation of specificity improvement when HSIA primer including fluorescent reporter, RNA sequence, and blocker is used in multiple looped-mediated isothermal amplification (LAMP) using fluorescence measurement real-time gene amplification equipment

In a method of amplifying a multiple-looped-mediated gene that amplifies two or more target genes, an individual gene amplification signal was confirmed by using only HSIA primer without a separate probe, and it was confirmed whether non-specific amplification by primer dimer was improved when using HSIA primer during multi-gene amplification.

The primers used were designed into sequences of N gene (SEQ ID NOs: 1, 2, 3, 4, 5, and 6) and RdRP gene (SEQ ID NOs: 9, 10, 11, 12, 13, and 14) sites using the sequence of SARS-CoV-2 sequence (GenebankID: NC_045512.2).

**[Table 3]**

| **Primer** | **SEQ ID NO.** | **SEQ ID NO: 5' -> 3'** |
|---|---|---|
| RdRP_F3 primer | SEQ ID NO: 9 | CCACTAGAGGAGCTACTGTA |
| RdRP_B3 primer | SEQ ID NO: 10 | TGACAAGCTACAACACGT |
| RdRP_FIP primer | SEQ ID NO: 11 | AGGTGAGGGTTTTCTACATCACTATATTGGAACAAGCAAATTCT ATGG |
| RdRP_BIP primer | SEQ ID NO: 12 | ATGGGTTGGGATTATCCTAAATGTGTGCGAGCAAGAACAAGTG |
| RdRP_LF primer | SEQ ID NO: 13 | CAGTTTTTAACATGTTGTGCCAACC |
| RdRP_LB primer | SEQ ID NO: 14 | GAGCCATGCCTAACATGCT |

The HSIA primers were designed for the N_FIP, N_BIP, RdRP_FIP, and RdRP_BIP primers. In order to confirm the fluorescence signal of each gene, thymine was designed to be an internal dT and then HEX dye was attached to the F2 sequence site of the N_FIP and N_BIP primer sequences. In addition, thymine was designed to be an internal dT and then Texas Red dye was attached to the F2 sequence site of the RdRP_FIP and RdRP_BIP primer sequences. At the 3' end of each primer, 3 bp of RNA sequence complementary to the SARS-CoV-2 sequence was added, and 4 bp of complementary DNA sequence was added so that the blocker was removed by RNase H when the target gene was present. Primers N_FIP_HSIA_dye, N_BIP_HSIA_dye, RdRP_FIP_HSIA_dye, and RdRP_BIP_HSIA_dye were designed with BHQ added at the 3' end to prevent primer dimer formation and to quench fluorescent dyes in the added sequence.

**[Table 4]**

| **Primer** | **SEQ ID NO.** | **SEQ ID NO: 5' -> 3' (r mark corresponds to RNA sequence)** |
|---|---|---|
| N_FIP_HSIA_dye primer | SEQ ID NO: 15 | TCTGGCCCAGTTCCTAGGTAGTGACGAAT (internal dT HEX) CGTGGTGGTGA rCrGrG TAAA-BHQ1 |
| N_BIP_HSIA_dye primer | SEQ ID NO: 16 | AGACGGCATCATATGGGTTGCACGGG  (internal  dT HEX) GCCAATGTGATCT rUrUrU GGTG-BHQ2 |
| RdRP_FIP_HSIA_dye primer | SEQ ID NO: 17 | AGGTGAGGGTTTTCTACATCACTATATT (internal dT Texas Red) GGAACAAGCAAATTCTATGG rUrGrG TTGG-BHQ1 |
| RdRP_BIP_HSIA_dye primer | SEQ ID NO: 18 | ATGGGTTGGGATTATCCTAAATGTG (internal dT Texas Red) GCGAGCAAGAACAAGTG rArGrG CCAT- BHQ2 |

1× isothermal amplification buffer 2 (NEB, USA), 0.1% v/v Tween 20, 8 mM MgSO₄, and 1.4 mM of each of dNTP, BST 3.0 polymerase 5U (NEB, USA), and SYBR green 1× (Biorad, USA) were used in common for each test. In order to confirm the performance of the conditions to which the HSIA primer was not applied, the N gene and RdRP primer combinations (SEQ ID NOs: 1, 2, 3, 4, 5, 6, 9, 10, 11, 12, 13 and 14) were used as 1.6 µM of FIP and BIP primers for each gene, 0.2 µM of F3 and B3 primers, and 0.4 µM of LF and LB primers, respectively.

In order to confirm the performance of the conditions to which the HSIA primer was applied, HSIA_dye primer combinations (SEQ ID NOs: 1, 2, 5, 6, 9, 10, 13, 14, 15, 16, 17 and 18) of N gene and RdRP were used as 1.6 µM of FIP and BIP primers for each gene, respectively, 0.2 µM of F3 and B3 primers, respectively, and 0.4 µM of LF and LB primers, respectively. In order to confirm whether the HSIA primer can remove the blocker and generate the fluorescence signal by RNase H in the multiple gene amplification, two conditions of non-addition and addition of RNase H were performed under the condition to which the HSIA primer was applied.

The positive signal was confirmed by using an Accuplex^{™} SARS-CoV-2 reference material (Seracare, USA) at a concentration of 1 × 10^{^5} copies/rxn, and DEPC-treated distilled water (Geneall, Korea) was added to the negative control group to confirm an amplification signal by a dimer. For the nucleic acid amplification conditions, after treatment by ABI7500 real-time PCR (Thermofisher, USA) equipment for 5 minutes at 55°C and 5 minutes at 70°C, the fluorescence of FAM, HEX, and Texas Red channels was measured every 1 minute at 60°C for 30 minutes.

Since SYBR green, which is an intercalating dye, binds double-stranded DNA, the amount of gene amplification by primer dimers can be confirmed in a positive standard material and a negative control group (DW) according to HSIA application by confirming the FAM fluorescence results obtained by measuring the amount of SYBR green.

FIG. 4 shows the effect of improving the specificity of the multiple looped-mediated isothermal gene amplification method and the ability to detect multiple targets when the HSIA primer is applied in the fluorescence measuring equipment, and in FIG. 4A, as a result of confirming the FAM fluorescence signal under the condition in which the HSIA primer is not applied, the amplification signal of the positive standard material was confirmed to be 7 to 8 minutes, and the amplification signal of the negative control group (DW) was confirmed to be 16 to 26 minutes. In FIG. 4B, as a result of confirming the FAM fluorescence signal under the conditions of applying the HSIA primer and not adding the RNase H, amplification signals for both positive and negative were not confirmed. In FIG. 4C, as a result of confirming the FAM fluorescence signal under the conditions of applying the HSIA primer and adding the RNase H, the amplification signal of the positive standard material was confirmed to be 6 to 7 minutes, and the amplification signal of the negative control group (DW) was not confirmed. Taken together with the results of FIGS. 4A, 4B, and 4C, it can be confirmed that gene amplification by dimers is reduced by HSIA primers in the multiple gene amplification, and normal amplification is performed in the positive standard material.

In FIGS. 4E and 4H, as a result of confirming fluorescence signals of the HEX and Texas Red channels under the conditions of applying the HSIA primer and not adding RNase H, neither positive nor negative amplification signals were confirmed. FIG. 4F shows that as a result of confirming the fluorescence signal of the HEX channel under the conditions of applying the HSIA primer and adding the RNase H, the amplification signal of the positive standard material was confirmed at 9 minutes and the amplification signal of the negative control group (DW) was not confirmed, and thus it was confirmed that the gene amplification and the fluorescence signal detection of the N gene were possible. In FIG. 4I, as a result of confirming the fluorescence signal of the Texas Red channel under the conditions of applying the HSIA primer and adding the RNase H, the amplification signal of the positive standard material was confirmed to be 8 to 9 minutes, and the amplification signal of the negative control group (DW) was not confirmed, and thus it was confirmed that gene amplification and fluorescence signal detection of the RdRP gene were possible. In addition, according to FIGS. 4D and 4G, it was confirmed that the HEX and Texas Red fluorescent signals were not detected when the HSIA primer was not applied, and it can be seen that the results of FIGS. 4F and 4G show that fluorescence was generated by amplifying the HSIA primer rather than the signal by SYBR green. Therefore, when FIGS. 4E, 4F, 4H, and 4I were summarized, it could be confirmed that when HSIA primer was applied, non-specific signal by primer dimer was reduced, and gene amplification by fluorescence was possible without a separate probe sequence.

### Example 3. Confirmation of specificity improvement when primer including blocker and RNA sequence is used in looped-mediated isothermal amplification (LAMP) using absorbance measuring isothermal gene amplification equipment

In absorbance measuring equipment, a primer dimer was formed, and it was examined whether specificity was improved when a HSIA primer using a primer including an RNA sequence and a blocker was applied to a primer sequence generated by a non-specific signal.

The primer used was designed as the sequence of the N gene site using the sequence of the SARS-CoV-2 sequence (GenebankID: NC_045512.2) (SEQ ID NOs: 1, 2, 3, 4, 5, and 6).

The HSIA primers were designed for the N_FIP and N_BIP primers. At the 3' end of N_FIP (SEQ ID NO: 3) and N_BIP (SEQ ID NO: 4), RNA sequence 3 bp complementary to the SARS-CoV-2 sequence was added, and complementary DNA sequence 4 bp was added so that the blocker was removed by RNase H when the target gene was present. In order to prevent dimer formation of the primer in the added sequence, a C3 blocker was added to the 3' end of N_FIP and N_BIP to design N_FIP_HSIA (SEQ ID NO: 7) and N_BIP_HSIA (SEQ ID NO: 8) primers.

When the HSIA primer was not applied, dNTP of each of 1 mM Tris-HCL, 10 mM (NH4)₂S0₄, 8 mM MgSO₄, 10 mM KCl, 0.1% v/v Tween 20, Cresol Red 16 mM, and 1.4 mM was used to confirm a difference between a positive amplification signal and a non-specific amplification signal in a real-time gene amplification device, and a combination of N gene and RdRP primer (SEQ ID NOs: 1, 2, 3, 4, 5, and 6) was used as 1.6 µM of FIP and BIP for each gene, 0.2 µM of F3 and B3, 0.4 µM of LF and LB, respectively, to confirm performance of a condition to which the HSIA primer was not applied. In order to confirm the performance of the conditions to which the HSIA primer was applied, a combination of N gene and RdRP HSIA primers (SEQ ID NOs: 1, 2, 5, 6, 7, and 8) was used as 1.6 µM of FIP and BIP for each gene, 0.2 µM of F3 and B3, and 0.4 µM of LF and LB, respectively. In order to confirm whether the HSIA primer can remove the blocker and generate the fluorescence signal by Hot start RNase H (1drop, Korea) in the multiple gene amplification, two conditions of non-addition of RNase H and addition of RNase H were performed under the condition of applying the HSIA primer.

The positive signal was confirmed by using an Accuplex^{™} SARS-CoV-2 reference material (Seracare, USA) at a concentration of 1 × 10^{^5} copies/rxn, and DEPC-treated distilled water (Geneall, Korea) was added to the negative control group to confirm an amplification signal by a dimer. For nucleic acid amplification conditions, after treatment with 1POT (1drop, Korea) equipment for 5 minutes at 55°C and 5 minutes at 70°C, absorbance was measured at 60°C for 30 minutes at a unit of 1 minute.

FIG. 5 shows the effect of improving the specificity of the looped-mediated isothermal gene amplification method when the HSIA primer was used in absorbance measuring equipment, and FIGS. 5A and 5B show test results when the HSIA primer was not applied, and in the case of a positive standard material, a change in absorbance was observed at 10 minutes, while in the case of a negative control group (DW), a change in absorbance was observed at 21 minutes, and thus non-specific amplification by the primer dimer was observed. In FIGS. 5C and 5D, as a result of the HSIA primer application and the RNase H non-addition test, both the positive amplification signal and the negative control amplification signal were not confirmed, and thus, when the blocker was not removed, the positive amplification signal and the non-specific amplification signal by the primer dimer were not confirmed. In FIGS. 5E and 5F, as a result of the HSIA primer application and the RNase H addition test, the amplification signal in the positive standard material was confirmed at 13 minutes, but the amplification signal in the negative control group was not confirmed. Thus, it can be seen from the results of FIGS. 5A, 5B, 5C, 5D, 5E, and 5F that non-specific amplification by the primer dimer is improved upon application of the HSIA primer, and the positive signal of the positive standard material is amplified.

### [Sequence list]

Sequence list electronic file attachment
(C:\KipoNet\NKEditor\appfile\1020220155260.xml)

## Claims

1. An inner primer modified for looped-mediated isothermal amplification (LAMP) of a target nucleic acid sequence,
wherein the inner primer is a forward inner primer (FIP), a backward inner primer (BIP), or a combination thereof,
wherein the FIP, the BIP, or the combination thereof includes an RNA sequence that is complementary to a template target nucleic acid sequence in a 3'-end direction and cleaved by Ribonuclease (RNase) and a blocker that inhibits gene amplification.

2. The inner primer for the LAMP of claim 1, wherein the RNA sequence cleaved by the RNase includes a reporter in a 5'-end direction.

3. The inner primer for the LAMP of claim 1, wherein the forward inner primer (FIP), the backward inner primer (BIP), or the combination thereof includes a DNA sequence that is complementary to the template target nucleic acid sequence in the 3'-end direction and recognized by the RNase.

4. A primer composition for looped-mediated isothermal amplification (LAMP), comprising:
the inner primer for the LAMP of any one of claims 1 to 3; and
a forward outer primer (F3) and a backward outer primer (B3) .

5. A kit for looped-mediated isothermal amplification (LAMP) comprising the composition of claim 4.

6. A method for detecting a target nucleic acid sequence, the method comprising:
(a) selecting a sequence of six regions of B3, B2, B1, F1c, F2c, and F3c or a sequence of six regions of F3, F2, F1, B1c, B2c, and B3c from 5' to 3'-end direction of the target nucleic acid sequence; and
(b) performing Looped-mediated isothermal amplification (LAMP) on a sample using the primer composition of claim 4 and a Ribonuclease (RNase).
